# EUROPÄISCHE PATENTANMELDUNG

(11) **EP 2 578 178 A2**
(43) Veröffentlichungstag der Anmeldung: **10.04.2013**
(21) Anmeldenummer: 12194818.6
(22) Anmeldetag: 28.04.2009
(51) Int. Cl.: A61B 19/00

(54) **Verfahren zur Ansteuerung einer behandlungsunterstützenden Software**

(62) Teilanmeldung aus: 09158956.4
(71) Anmelder: BrainLAB AG, 85622 Feldkirchen (DE)
(72) Erfinder: Lechner, Christian, 82287 Jesenwang (DE); Plassky, Norman, 99099 Erfurt (DE); Millahn, Manuel, 80799 München (DE); Christian, Georg, 82544 Egling (DE)
(74) Vertreter: Schwabe - Sandmair - Marx

(57) **Zusammenfassung**

Die Erfindung betrifft ein Verfahren zur Ansteuerung einer behandlungsunterstützenden medizintechnischen Software mittels eines Signal-Senders (5), der an einem medizinischen Instrument (10) angeordnet ist, wobei das Instrument (10) mittels eines medizintechnischen Trackingsystems (31) positionell erfasst wird, und wobei dem vom Sender (5) abgegebenen Signal je nach Position des Instruments (1) von der Software eine unterschiedliche Bedeutung zugeordnet wird.

## Beschreibung

Die Erfindung betrifft ein Verfahren zur Ansteuerung einer behandlungsunterstützenden medizintechnischen Software mittels eines Signal-Senders (5), der an einem medizinischen Instrument (10) angeordnet ist, wobei das Instrument (10) mittels eines medizintechnischen Trackingsystems (31) positionell erfasst wird, und wobei dem vom Sender (5) abgegebenen Signal je nach Position des Instruments (1) von der Software eine unterschiedliche Bedeutung zugeordnet wird.

Die vorliegende Erfindung betrifft ein Verfahren zur Ansteuerung einer behandlungsunterstützenden medizintechnischen Software mittels eines Signal-Senders, der an einem medizinischen Instrument angeordnet ist. Das medizinische Instrument wird positionell mittels eines medizintechnischen Trackingsystems erfasst, und dem vom Sender abgegebenen Signal wird je nach Position des Instruments von der Software eine unterschiedliche Bedeutung zugeordnet.

Dieses System, bei dem Instrumente zum Einsatz kommen können, wie sie hierin beschrieben und beansprucht werden, ermöglicht einerseits eine breite Kommunikationsbasis und das Übertragen von unterschiedlichen, auch komplizierten Steuerungsbefehlen, andererseits können dadurch Signalsendereinheiten sehr einfach gehalten werden. Die Erfindung sieht nämlich vor, dass nicht unbedingt das Signal geändert werden muss, um unterschiedliche Befehle weiterzuleiten, sondern einfach nur die Position des Instruments geändert werden kann, an welchem der Signalgeber sich gerade befindet. Weil solche Instrumente, beispielsweise Pointer, vom behandelnden Arzt relativ einfach in gewisse Positionen bringbar sind, bietet die vorliegende Erfindung die Möglichkeit, eine Fülle von Signalen mit einer einfachen Instrumentenausstattung bzw. einem einfachen Sender zu übertragen.

In spezieller Ausgestaltung des erfindungsgemäßen Verfahrens wird im Erfassungsbereichs des Trackingsystems, insbesondere im Umfeld bzw. in der Umgebung einer Trackingreferenz mindestens ein Raumbereich definiert, wobei das vom Sender abgegebene Signal von der Software eine bestimmte Bedeutung erhält, wenn das Instrument mit diesem Raumbereich interagiert, insbesondere die Spitze (oder ein bestimmter Teil) des Instruments sich in diesem Raumbereich befindet.

Es besteht ferner die Möglichkeit, mittels des Signal-Senders ein Instrumenten-Identifizierungs-Signal oder einen Code zu senden, was die Initialisierung (bzw. Kalibrierung) der Instrumente, beispielsweise für ein operationsbegleitendes medizintechnisches Navigationssystem, sehr vereinfachen kann.

Ein weiterer Aspekt der vorliegenden Erfindung betrifft die Erfindung ein Programm, das, wenn es auf einem Computer läuft oder in einem Computer geladen ist, den Computer veranlasst, ein Verfahren durchzuführen, wie es hierin beschrieben wird, sowie ein Computerprogrammspeichermedium mit einem solchen Programm.

Medizinische Instrumente, wie sie im Rahmen der vorliegenden Erfindung bereitgestellt werden, können zusätzlich zu ihrem eigentlichen Instrumentenzweck noch zur Ansteuerung behandlungsunterstützender Software verwendet werden. In der computergestützten Chirurgie werden dem Behandelnden - meist von einem Navigations- bzw. Trackingsystem im Operationssaal - unterschiedliche Bildausgaben zur Verfügung gestellt. Diese Bildausgaben zeigen Patientendatensätze oder zumindest Teile davon, also z.B. dreidimensionale oder Schnittwiedergaben von Patientenkörperteilen. Die Patientendatensätze können entweder durch bildgebende Verfahren wie z.B. CT- oder MR-Tomographie, Röntgen, Ultraschall oder Fluoroskopie, oder durch bildlose Verfahren, wie das Abgreifen einer Knochenoberfläche mittels eines registrierten Zeigerwerkzeugs oder mit Laserabtastung erzeugt werden. Ferner können im Rahmen der Bildunterstützung beispielsweise Instrumente oder Behandlungseinrichtungen im Positionsverhältnis zu den Patientendaten gezeigt werden, um so dem Behandelnden eine visuelle Unterstützung zu geben. Je nach Behandlungsfortschritt wird es oft notwendig, einen speziellen Teil der Softwareunterstützung auf der Bildschirmausgabe anzuzeigen, nämlich den Teil mit den Funktionen, die für den momentanen Behandlungsschritt gerade erforderlich sind. Man könnte auch sagen, dass die Software aus verschiedenen "Seiten" besteht, die im Laufe der Behandlung durchwechseln. Oft ist es notwendig, während der Behandlung bestimmte Software-Seiten anzuwählen. Neben der oben angesprochenen Lösung wird dies noch oft mittels eines Eingabegerätes wie einer Maus oder einer Tastatur oder mittels eines berührungsempfindlichen Bildschirms bewerkstelligt. Auch Fußschalter, virtuelle Tastaturen oder Sprachsteuerungen sind bekannt. Andere "Kommunikationsmittel" sind beispielsweise spezielle Bewegungen von getrackten Instrumenten, beispielsweise das Schwenken um einen Festpunkt ("Pivoting").

Vor diesem Hintergrund ist es die Aufgabe der vorliegenden Erfindung, das Senden von Signalen zur Kommunikation und Steuerung in Verbindung mit medizinischen Instrumenten zu optimieren.

Diese Aufgabe wird erfindungsgemäß durch ein Verfahren gemäß dem Anspruch 1 gelöst. Die Unteransprüche definieren bevorzugte Ausführungsformen der Erfindung.

Die im Rahmen der vorliegenden Erfindung bereitgestellten Instrumente zeichnet sich dadurch aus, dass die Sendereinheit eine außen am Instrument befestigbare Einheit ist. Mit anderen Worten wird eine am Instrument ansetzbare Sendereinheit bereitgestellt, was die Ausstattung von Instrumenten mit der Sender-Funktionalität stark vereinfacht. Es ist möglich, Sendereinheiten bereitzustellen, die leicht bzw. mit einfachen Maßnahmen an herkömmlichen Instrumenten befestigbar sind, so dass die Notwendigkeit der Bereitstellung spezieller und damit teurer Instrumente entfällt. Die Erfindung beruht unter anderem auf der Erkenntnis, dass die Sterilität solcher Instrumente nicht unbedingt gefährdet ist, wenn außen am Instrument ansetzbare Sendereinheiten verwendet werden, und der Grund hierfür liegt darin, dass auch solche Sendereinheiten in ihrer Gesamtheit vorsterilisiert, insbesondere steril verpackt bereitgestellt werden können. Damit wird eine kostengünstige Lösung ohne Sterilitätsprobleme möglich, speziell können die Probleme umgangen werden, die das Heißdampfsterilisieren das in der Praxis am meisten eingesetzt wird, bei elektronischen Bauteilen und Energieversorgungselementen verursacht.

Bei einer Ausführungsform der vorliegenden Erfindung ist am Instrument ein positionell definierter Fixierungsabschnitt für die Sendereinheit vorgesehen, während andererseits auch die Sendereinheit einen Befestigungsabschnitt aufweisen kann, der am Umfang des Instruments, insbesondere an einem solchen Fixierungsabschnitt befestigbar ist.

Um die Handhabbarkeit zu optimieren, kann die Sendereinheit mittels eines lösbaren Schnellverschlusses befestigbar sein, insbesondere mittels einer Schnapp- und/oder Steckverbindung. Gemäß einer Ausführungsvariante ist sie mit einem am Umfang des Instruments angeordneten Fixierungselement befestigbar, insbesondere mit einem abnehmbaren und wieder anbringbaren Instrumententeil, speziell einem am Instrument anbringbaren Referenzmarker.

Die Sendereinheit kann verschiedene funktionelle Elemente enthalten, unter anderem einen optischen Sender, speziell eine LED, insbesondere eine Infrarot-LED und einen Betätigungsschalter, speziell einen Berührungsschalter. Zusätzlich kann noch ein optischer Statussignalgeber, insbesondere eine Statussignal-LED vorgesehen werden.

Es besteht die Möglichkeit, das medizinische Instrument so auszugestalten, dass die Sendereinheit eine integrierte Energieversorgung für den Sender aufweist, insbesondere eine Batterie, speziell eine Knopfzellenbatterie.

Bei einer besonders bevorzugten Ausführungsform wird die Sendereinheit als Einwegartikel bereitgestellt, speziell als steril vorverpackter Einwegartikel, der beispielsweise in einer Aufreißpackung vor Ort im Operationssaal bereitgestellt werden kann. Heutige Herstellungsmethoden lassen es zu, kostengünstig solcherlei Wegwerfartikel beispielsweise im Wesentlichen oder hauptsächlich (überwiegend) aus einem Kunststoffmaterial zu produzieren, und in Operationssälen, wo die Sterilität eine äußerst wichtige Bedingung ist, ist eine solche Praxis durchaus gerechtfertigt und wird vielfältig schon angewandt.

Gemäß einem anderen Aspekt der vorliegenden Erfindung betrifft diese ein Instrumentensystem mit mehreren Instrumenten, wie sie hierin in verschiedenen Ausführungsformen beschrieben werden, wobei die Instrumente standardisierte Fixierungsabschnitte aufweisen, welche die gleiche Sendereinheit bzw. eine Sendereinheit mit gleichen, standardisierten Befestigungsabschnitten befestigen können, und mit mindestens einer separaten Sendereinheit. Diese Ausführungsvariante macht den universellen Einsatz der Erfindung mit vielen verschiedenen Instrumenten möglich.

Ein im Rahmen der vorliegenden Erfindung bereitgestelltes Instrument wird im Weiteren anhand einer Ausführungsform näher erläutert. Die Erfindung kann alle hierin beschriebenen Merkmale einzeln sowie in jedweder Kombination umfassen und insbesondere auch als Verwendung eines separaten, außen am Instrument ansetzbaren Signalsenders für die Softwaresteuerung angesehen werden. In den beiliegenden Zeichnungen zeigen:
- Fig. 1 bis 3: eine Ausführungsform einer Sendereinheit;
- Fig. 4 und 5: die Ausstattung eines medizinischen Instruments mit einer Sendereinheit; und
- Fig. 6 und 7: zwei Ausführungsformen für die Signalübertragung in Abhängigkeit von der Instrumentenposition.

In den beiliegenden Zeichnungen wird eine Ausführungsform einer Sendereinheit mit dem Bezugszeichen 1 gekennzeichnet. Sie weist einen Körper mit einem Befestigungsabschnitt 2 und einem Fortsatz bzw. LED-Träger 3 auf, der eine Signal-LED 5 und eine Status-LED 4 aufweist. Gegenüber dem Träger 3 ist der Betätigungsschalter 6, hier ein Berührungsschalter, am Befestigungsabschnitt 2 angebracht. Der Schalter 6 kann jedweder Schalter sein, beispielsweise auch ein Druckschalter.

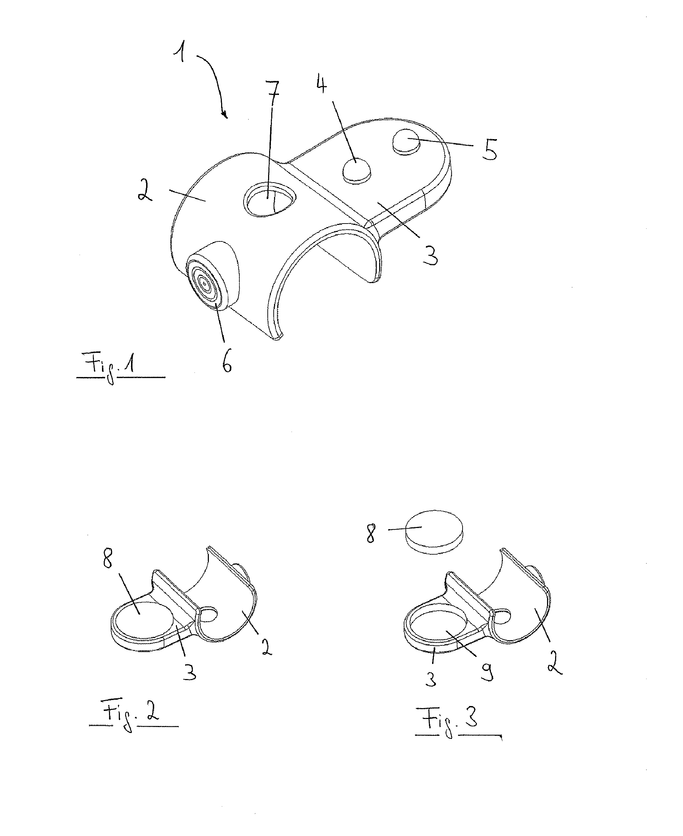

Im unteren Teil des Trägers 3 ist eine Aussparung 9 für eine Knopfzellenbatterie 8 vorgesehen, welche die Energieversorgung für die Signalabstrahlung durch die LEDs 4, 5 bereitstellt. Im oberen Teil des Befestigungsabschnitts 2 befindet sich ein Durchgang 7, hier ein kreisförmiges Loch. Die vorgenannten Bauteilbezeichnungen sind insbesondere den Figuren 1 bis 3 entnehmbar.

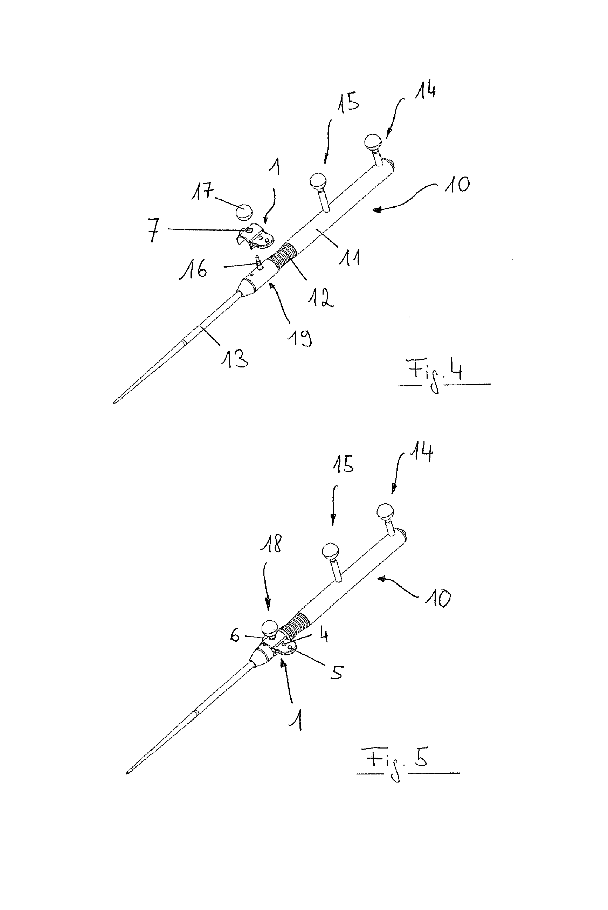

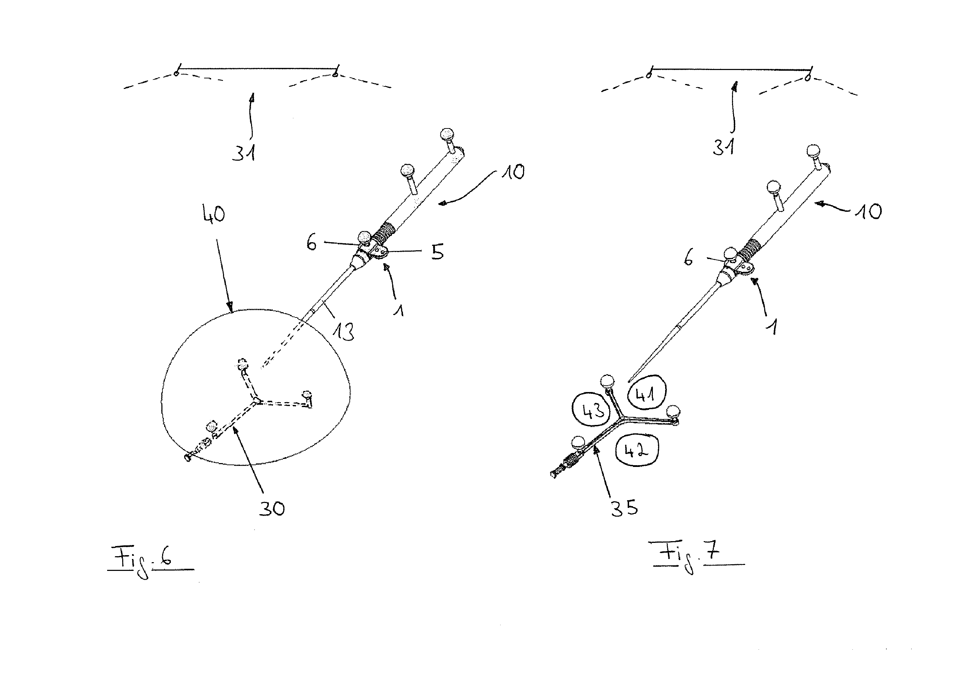

In den Figuren 4 bis 7 ist ein Instrument 10 gezeigt, das einen Instrumentengriff 11 und eine Spitze 13 umfasst und als Pointer bzw. Zeigegerät ausgestaltet ist. Der Griff hat einen geriffelten Abschnitt 12, an dem er sicher gehalten werden kann, und nach oben ragen aus dem Griff 3 Referenzmarker 14, 15 und 18 hervor, die als Trackingmarker für ein medizintechnisches Trackingsystem 31 dienen, das zwei Trackingkameras aufweist, die schematisch in den Figuren 6 und 7 gezeigt, aber nicht separat bezeichnet sind. Der vorderste Trackingmarker 18 ist in Figur 4 detaillierter gezeigt, und er weist am Fixierungsabschnitt 19 den herausragenden Markerhalter 16 sowie die Markerkugel 17 auf. Wie den Figuren 4 und 5 entnehmbar ist, wird bei dieser Ausführungsform die Sendereinheit 1 mit dem Durchgangsloch 7 auf den Markerhalter 16 und dann auf den Fixierungsabschnitt 19 aufgesteckt und durch das Aufschrauben der Markerkugel 17 fixiert bzw. gegen unbeabsichtigtes Verlieren gesichert.

In den Figuren 6 und 7 sind noch verschiedene definierte Raumbereiche 40, 41, 42 und 43 gezeigt, die um Trackingreferenzen 30 bzw. 35 herum gebildet bzw. definiert werden. Die weitere Beschreibung der Erfindung stützt sich nun auf die genannten Figuren und Bezeichnungen.

Ebenso wie jedes Standard-Navigationsinstrument mit passiver Trackingtechnologie hat auch das Instrument 10 Reflektionsmarker 14, 15 und 18, und diese Marker bzw. ihre reflektierenden Kugeln (z.B. 17) reflektieren ausgestrahltes Infrarotlicht und werden dabei vom Trackingsystem 31 positionell erfasst. Herkömmliche Navigationssysteme verwenden diese Positionsdaten als dreidimensionale Positionsinformationen für bestimmte Markergeometrien und ordnen diese bestimmten Instrumenten zu, so dass im Navigationssystem beispielsweise immer bekannt ist, wo sich das vordere Ende der Spitze 13 des Instruments 10 befindet.

Die Sendereinheit 1 gemäß der vorliegenden Erfindung, die als aktive Eingabevorrichtung zur Softwaresteuerung angesehen werden kann, wird wie in den Figuren 4 und 5 dargestellt und wie oben schon beschrieben an einem Instrumentenmarker 18 am Instrument 10 befestigt. In dieser Position ist der Schalter 6 gut mit einem Finger erreichbar; wenn er betätigt wird, strahlt die Infrarot-LED 5 ein Infrarot-Lichtsignal zur Softwaresteuerung ab, das z.B. vom Trackingsystem 31 erfasst wird, und die LED 4 strahlt ein sichtbares Lichtsignal als Bestätigungs- oder Statussignal ab, damit der Benutzer weiß, dass das unsichtbare Infrarot-Signal der LED 5 auch tatsächlich gesendet worden ist. Die Stromversorgung hierfür erfolgt, wie schon oben angedeutet, durch die Knopfzelle 8 im Batteriehalter 9.

Die hier als Einwegartikel aus Kunststoff gefertigte Sendereinheit 1 ist vorteilhafterweise symmetrisch anbringbar aufgebaut, so dass sie an dem Instrument universal für rechts- oder linkshändige Bedienung angebracht werden kann, also mit der Ausrichtung des Trägerfortsatzes 3 in die eine oder andere Richtung des Instrumentenkörpers, weg von dessen Längsachse. Bei der bevorzugten Ausführungsform ist die Sendereinheit 1 an einer definierten Position des navigierten Instruments 10 angebracht, nämlich am Fixierungsabschnitt 19, so dass die Software den Positionsbereich des IR-LED-Trägersignals erkennen kann, der mit den geometrischen Informationen über das Instrument 10 im Navigationssystem abgespeichert ist.

Wenn der Betätigungsschalter 6 betätigt wird, wird das IR-LED-Signal der Diode 5 aktiviert. Die LED 5 wird während dieses "aktiven Modus" durch die Trackingkameras des Trackingsystems 31 getrackt bzw. verfolgt und das ausgesendete Signal wird verwendet, um eine Softwarefunktionalität des Navigationssystems, beispielsweise als Bestätigungssignal anzusteuern.

Die Positionsgenauigkeit der IR-LED 5 ist dabei weniger wichtig, weil nur die genauer positionierten passiven Marker 14, 15 und 18 die Instrumentengeometrie referenzieren. Aus diesem Grund kann die Sendereinheit sehr einfach, billig und als steriler Einweg- oder Wegwerfartikel ausgebildet werden. Aktive Triggersignale könnten bei einer weiteren Ausführungsform verwendet werden, um Registrierungspunkte auf Objekten, z.B. Knochenoberflächen, zu digitalisieren, ohne dass zusätzliche Trigger-Bewegungen oder andere Trigger-Signale erzeugt werden müssen.

Was die Anwendbarkeit angeht, können die offenbarten aktiven Einweg-Sendereinheiten universal in Kombination mit allen möglichen existierenden passiven Instrumenten und Zeigegeräten (Pointer) verwendet werden und somit schon existierende passive Pointergeräte mit aktiven Steuerungsfunktionalitäten ausstatten bzw. verbessern. Die Sendereinheiten könnten außerdem für andere passive navigierte Instrumente kompatibel ausgestaltet werden, beispielsweise für Bohrerführungen, Schneidblöcke, Aalen, Räumwerkzeuge usw., wobei sie an diesen Instrumenten ebenfalls (als Einwegartikel) befestigt und nach der Verwendung wieder abgenommen würden. Wenn solche Instrumentensätze bereitgestellt werden, könnten sie speziell zugeordnete und ausgestaltete Schnittstellen zur Anbringung der Universal-Einweg-Sendereinheiten haben. Weitere technische Merkmale können integriert werden, wie zusätzliche IR-LEDs, kabellose RF-Kommunikationsmodule oder mehrere Steuerungsknöpfe, was die Funktionalität noch steigern würde, insbesondere auch in Hinsicht auf die Software-Interaktion für Standard-Passivinstrumente.

Unter spezieller Bezugnahme auf die Figuren 6 und 7 soll nun der Erfindungsaspekt näher erläutert werden, der als berührungslose Software-Workflow-Ansteuerung bezeichnet werden kann. Während der medizintechnischen Navigation werden für gewöhnlich Referenzanordnungen 30, 35 bereitgestellt und an Patienten befestigt, um dessen Bewegungen nachzuvollziehen und in die Navigation integrieren zu können. Anhand dieser (oder auch anderer bereitstehender) Referenzgeometrien 30, 35 lassen sich nunmehr vordefinierte, virtuelle Bereiche im Raum festlegen, die zur Benutzung des Instruments als Fernsteuerung dienen können. Wenn ein navigiertes Instrument beispielsweise mit seinem spitzen Ende in einen solchen Fernsteuerungsbereich, beispielsweise den Bereich 40 um die Referenzanordnung 30 in Figur 6, eingebracht wird, wird ein bestimmter "Fernsteuerungsmodus" für die Software gestartet.

Bei einer "Ein-Knopf"-Steuerung kann der Verwender dann Funktionalitäten der aktiven Sendereinheit steuern, um einfache Befehle wie "Vor", "Zurück" oder "Auswahl" zu übermitteln. So lassen sich z.B. kurzen Signalen (weniger als 0,5 sec) "Vor"-Funktionen zuordnen, während längere Betätigungen des Schalters 6 (mehr als eine Sekunde) als "Zurück"-Befehl eingestuft und vordefiniert werden können, wenn sich die Spitze 13 des Pointer Instruments 10 im virtuellen Bereich 40 um die Referenzanordnung 30 befindet. Wenn nun eine andere Funktion, beispielsweise eine "Auswahl"-Funktion notwendig wird, kann der Benutzer das vordere Ende der Spitze 13 aus dem virtuellen Bereich 40 um die Referenzanordnung 30 heraus nehmen, und wenn vom Tracking- und Navigationssystem festgestellt wird, dass dieses Spitzenende sich nicht im Bereich 40 befindet, wird der Betätigung des Schalters 6 ein anderer Befehl zugeordnet, beispielsweise ein "Auswahl"-Befehl. Abfolgende Klicks auf die aktive Eingabevorrichtung 1 würden dann beispielsweise Referenzpunkte auf Patientenkörperteilen bestätigen, auswählen, aktivieren oder digitalisieren. Mit einem "anhaltenden" Aktivieren des Knopfes bzw. des Signalgebers können außerdem multiple Oberflächen registriert werden, wobei die Spitze des Pointer-Instruments über die zu registrierende Oberfläche geführt wird und die Software dafür sorgt, dass Punkte nicht mehrfach aufgenommen werden.

Die erfindungsgemäßen Fernsteuerungsbereiche 40 befinden sich in einem frei definierten und definierbaren Raum um die Referenzanordnung 30.

Eine weitere in Figur 7 gezeigte Ausführungsform sieht vor, den Fernsteuerungsbereich in drei freie Bereiche 41, 42 und 43 zwischen den Armen des Referenzsterns 35 zu unterteilen. Jeder Bereich hat dann eine andere Funktionalität, wenn die Pointerspitze in ihn eingebracht wird, und entsprechende Funktionalitäten können durch das Drücken des Betätigungsschalters 6 abgerufen werden. Dabei kann die Spitze des Pointers frei im Raum bleiben und muss den Referenzstern 35 nicht an vorbestimmten oder vordefinierten Stellen berühren, so dass Referenzverschiebungen und dadurch Registrierungsungenauigkeiten vermieden werden.

Mit ihren Ausführungsformen ermöglicht die vorliegende Erfindung also eine berührungslose, kabellose und sehr komfortable Interaktion des Behandelnden mit einer Software eines Navigationssystems. Der Behandelnde steuert die Systemfunktionalitäten durch die Verwendung eines navigierten Instruments im sterilen Feld. Indem dem passiven navigierten Instrument eine aktive Interaktionsvorrichtung zugeordnet wird, werden die Verfahrensmöglichkeiten erweitert und gestatten eine größere Vielfalt an Steuerungseingaben (z.B. bei der Registrierung), ohne dass die Bewegungen oder separate Auswahl- oder Bestätigungs-Ansteuerungen durchgeführt werden müssen. Die Sendereinheit kann als Einwegeinheit und aktives Zusatzelement an existierende passive, navigierte Instrumente angesetzt werden (z.B. als Clip), und damit lassen sich Probleme vermeiden, die aufgrund von integrierter Elektronik entstehen, wenn Wärme und Dampf bei Sterilisierungen zum Einsatz kommen.

Die Vorteile der allgemeinen und verschiedener spezieller Ausführungsformen der Erfindung können noch wie folgt aufgelistet werden:
- Eine intuitive Interaktion zwischen Anwender und Software/Computersystem ist möglich;
- Eine komfortablere, leichtere und schnellere Registrierung von Objekten im Navigationssystem kann erfolgen;
- Ein Abrutschen bei Objektregistrierungen, z.B. Knochenregistrierungen ist vermeidbar, weil Bestätigungsbewegungen vermieden werden (Pivoting);
- Eine optimale Referenzanordnungs-Ausrichtung zur bestmöglichen Wiedererkennung/Genauigkeit bei der Registrierung wird ermöglicht (keine Schwenkbewegungen durch Bereiche mit eingeschränkter Sichtbarkeit notwendig);
- Verwender-Flexibilität wird bereitgestellt;
- Ein einfaches Instrumenten-Design ist möglich;
- Es besteht kein Bedarf an einer Autoklavierung, so dass Schäden an elektronischen Bauteilen und Energieversorgungselementen vermieden werden können;
- Kompatibilität mit existierenden passiven Instrumenten, wie z.B. Pointern, besteht; und
- Jedwedes passive Instrument kann mit zusätzlichen aktiven Funktionalitäten erweitert werden.

## Patentansprüche

1. Verfahren zur Ansteuerung einer behandlungsunterstützenden medizintechnischen Software mittels eines Signal-Senders (5), der an einem medizinischen Instrument (10) angeordnet ist, wobei das Instrument (10) mittels eines medizintechnischen Trackingsystems (31) positionell erfasst wird, **dadurch gekennzeichnet, dass** dem vom Sender (5) abgegebenen Signal je nach Position des Instruments (1) von der Software eine unterschiedliche Bedeutung zugeordnet wird.

2. Verfahren nach Anspruch 1, bei dem im Erfassungsbereich des Trackingsystems (31), insbesondere im Umfeld bzw. in der Umgebung einer Trackingreferenz (30, 35) mindestens ein Raumbereich (40, 41, 42, 43) definiert wird, wobei das vom Sender (5) abgegebenen Signal von der Software eine bestimmte Bedeutung erhält, wenn das Instrument (10) mit diesem Raumbereich interagiert, insbesondere die Spitze des Instruments sich in diesem Raumbereich befindet.

3. Verfahren nach Anspruch 1 oder 2, bei dem folgendes Instrument zur Software-Ansteuerung verwendet wird: Medizinisches Instrument (10) mit einem Signalsender (5) und mindestens einer Betätigungseinrichtung (6) zur Betätigung des Senders (5), der an einer vom Instrument separaten Sendereinheit (1) vorgesehen ist, wobei die Sendereinheit (1) eine außen am Instrument (10) befestigbare Einheit ist.

4. Verfahren nach Anspruch 3, wobei am Instrument (10) ein positionell definierter Fixierungsabschnitt (19) für die Sendereinheit (1) vorgesehen ist.

5. Verfahren nach einem der Ansprüche 3 oder 4, wobei die Sendereinheit (1) einen Befestigungsabschnitt (2) aufweist, der am Umfang des Instruments (10), insbesondere am Fixierungsabschnitt (19), befestigbar ist.

6. Verfahren nach einem der Ansprüche 3 bis 5, wobei die Sendereinheit (1) mittels eines lösbaren Schnellverschlusses befestigbar ist, insbesondere mittels einer Schnapp- und/oder Steckverbindung.

7. Verfahren nach einem der Ansprüche 3 bis 6, wobei die Sendereinheit (1) mit einem am Umfang des Instruments (10) angeordneten Fixierungselement befestigbar ist, insbesondere mit einem abnehmbaren und wieder anbringbaren Instrumententeil, speziell einem am Instrument (10) anbringbaren Referenzmarker (17).

8. Verfahren nach einem der Ansprüche 3 bis 7, wobei die Sendereinheit (1) einen optischen Sender, speziell eine LED, insbesondere eine Infrarot-LED (5) und einen Betätigungsschalter, speziell einen Berührungsschalter (6) aufweist.

9. Verfahren nach einem der Ansprüche 3 bis 8, wobei die Sendereinheit (1) einen optischen Statussignalgeber, insbesondere eine Statussignal-LED aufweist.

10. Verfahren nach einem der Ansprüche 3 bis 9, wobei die Sendereinheit (1) eine integrierte Energieversorgung für den Sender (1) aufweist, insbesondere eine Batterie, speziell eine Knopfzellenbatterie (8).

11. Verfahren nach einem der Ansprüche 3 bis 10, wobei die Sendereinheit (1) ein Einwegartikel ist.

12. Verfahren, bei dem zur Software-Ansteuerung ein Instrumentensystem mit mehreren Instrumenten gemäß einem der Ansprüche 3 bis 11 verwendet wird, die standardisierte Fixierungsabschnitte (19) aufweisen, welche die gleiche Sendereinheit (1) bzw. Sendereinheiten mit gleichen, standardisierten Befestigungsabschnitten (2) befestigen können, und mit mindestens einer separaten Sendereinheit (1).

13. Verfahren nach einem der Ansprüche 1 bis 12, bei dem mittels des Signal-Senders ein Instrumenten-Identifizierungs-Code gesendet wird.

14. Programm, das, wenn es auf einem Computer läuft oder in einem Computer geladen ist, den Computer veranlasst, ein Verfahren gemäß einem der Ansprüche 1 bis 13 durchzuführen.

15. Computerprogramm-Speichermedium, das ein Programm nach Anspruch 14 aufweist.
